# EUROPEAN PATENT SPECIFICATION

(11) **EP 4 347 552 B1**
(45) Date of publication and mention of the grant of the patent: **21.05.2025**
(21) Application number: 22730769.1
(22) Date of filing: 23.05.2022
(51) Int. Cl.: C07C 209/48, C07C 209/84, C07C 211/36, C07C 209/52

(54) **METHOD FOR MANUFACTURE OF ISOPHORONEDIAMINE**
VERFAHREN ZUR HERSTELLUNG VON ISOPHORONDIAMIN
PROCÉDÉ DE FABRICATION D'ISOPHORONEDIAMINE

(30) Priority: 02.06.2021 EP 21177453
(43) Date of publication of application: 10.04.2024
(73) Proprietor: BASF SE, 67056 Ludwigshafen am Rhein (DE)
(72) Inventor: SCHLODERER, Martin, 67056 Ludwigshafen (DE); KRAUSE, Alfred, 67056 Ludwigshafen (DE); HERRMANN, Andreas Edgar, 67056 Ludwigshafen (DE); GOUVERNEUR, Hannes Ferdinand, 67056 Ludwigshafen (DE)
(74) Representative: BASF IP Association
(86) International application number: PCT/EP2022/063866
(87) International publication number: WO 2022/253610

(56) References cited:
- EP-A1- 3 354 624
- EP-A2- 1 529 028
- EP-B1- 1 529 028

## Description

The present invention relates to a process for the manufacture of isophoronediamine (IPDA).

IPDA is used as a starting product for preparing isophorone diisocyanate (IPDI), an isocyanate component for polyurethane systems, as an amine component for polyamides and as a hardener for epoxy resins.

IPDA is usually prepared in a multistage process starting from isophorone (IP). In a first step, hydrogen cyanide (HCN) is added to IP to obtain the corresponding isophoronenitrile (IPN). In a further step, IPN is converted to IPDA by converting the carbonyl group of IPN to an amino group and the nitrile group to an aminomethyl group in the presence of ammonia, hydrogen and hydrogenation catalysts. The second step can be divided into further steps, in which the carbonyl group of IPN is first converted with ammonia (NH3) to the corresponding isophoroneni-trilimine (IPNI) in the presence of an imination catalyst. In a subsequent step, IPNI is then hydrogenated in the presence of a hydrogenation catalyst to obtain IPDA.

IPDA is usually worked-up in a series of distillation steps.

EP3235805 (Evonik) discloses a process for the purification of IDPA by separation of ammonia and hydrogen in a first section to obtain crude-IPDA and further purification of the crude IPDA in a series of distillation steps. Crude-IPDA is fed to a vacuum distillation column where impurities with a boiling point lower than IPDA are separated. The condensate obtained at the top of the column is condensed in a series of condensers. The partial condensate obtained in the first partial condenser is recycled as reflux into the column. The condensate obtained in the second condenser comprises an aqueous and an organic fraction. These fractions are separated in a phase separator into an aqueous and an organic phase which are channeled out of the process. The bottoms from the first column are further separated in a second vacuum column to obtain purified IPDA as a top product and a bottom product comprising impurities with a higher boiling point than IPDA.

A similar process is disclosed in EP3275858 (Evonik). EP3275858 comprises an additional partial condenser in the second distillation column used for the final purification of IPDA.

EP3248960 (Evonik) discloses the use of a dividing wall column for the purification of crude IPDA. In this process, the low boiling components, comprising an aqueous and an organic phase, are removed from the top of the dividing wall column. Pure IPDA is withdrawn as a side fraction and the higher boiling impurities are removed from the bottom of the column.

The Evonik patents disclose that an aqueous phase comprising impurities and having a boiling point lower than IPDA is obtained in the first vacuum column. It is reported that this fraction is at least partially channeled out of the process. However, the disclosures appear to be silent relating to the work-up or use of the fraction which is channeled out of the process.

EP1529028 (BASF) discloses a process for the purification of IPDA wherein hydrogen and ammonia are removed to obtain a so-called crude IPDA. Crude IPDA is separated in one or more conventional or dividing wall columns into a least five fractions. The first fraction being a fraction of organic impurities having a lower boiling point than trans-IPDA, the second fraction being an aqueous fraction of impurities having a lower boiling point than trans-IPDA, the third fraction is a fraction of impurities having a higher boiling point than cis-IPDA, a fourth fraction comprising IPDA having a cis-trans-ratio (CTR) of 73:27 and more and a fifth fraction comprising IPDA having a CTR of 66:34 or less. EP1529028 discloses that the second fraction comprising aqueous impurities requires wastewater treatment. However, details of the waste-water treatment do not seem to be disclosed.

EP3354624 (Evonik) discloses a method for treating wastewater from isophorone (IP), isophoronenitrile (IPN) and isophoronediamine (IPDA) production. A main focus of the disclosure is the removal of IPN from wastewater obtained in the production of IPN by the addition of HCN to IP. EP3354624 teaches the removal of IPN impurities in the wastewater by an alkaline hydrolysis of IPN to IP and HCN in a specific temperature and pH-range. EP3354624 further discloses the treatment of combined wastewaters from the alkaline hydrolysis of IPN together with the wastewater from IPDA production. The HCN-comprising wastewater is first treated in an oxidation step before it is fed into a biological waste-water treatment plant. It is reported that the oxidation step leads to a reduction of the chemical oxygen demand in the biological waste-water plant by 50% or more. It is further reported that oxidation results in an oxidation of residual HCN or salts thereof to carbon dioxide.

Whereas EP3354624 discloses that HCN-comprising wastewater from IPDA production can be treated together with the wastewater from the alkaline hydrolysis of the wastewater from IPN production by chemical oxidation and biological treatment, it does not seem to disclose how other wastewater streams from IPDA production comprising other impurities than HCN are to be treated or how to treat streams from IPDA production without combining them with wastewater streams from upstream operations.

The object of the invention was to provide a method for the production of IPDA which allows for efficient treatment of the aqueous fraction obtained by the removal of the components having a boiling point lower than IPDA.

It was found that the aqueous fraction obtained in the separation of low boiling components from IPDA comprises organic impurities which are difficult to degrade in a conventional biological wastewater treatment plant.

It was therefore an object of the invention to provide for a wastewater treatment allowing the removal of components which are difficult to degrade in a biological wastewater treatment plant and to lower the chemical oxygen demand required in the treatment of such fractions. A further object of the present invention was to provide for a method for the production of IPDA allowing to meet high environmental standards and regulations.

The object of the present invention was achieved by
a method for the production of IPDA comprising the steps of:
I. converting IPN with ammonia and hydrogen to IPDA,
II. removing hydrogen and/or ammonia from the effluent from step I
III. separating the effluent from step II in one or more steps into
   (i) a fraction comprising an organic phase having a boiling point lower than IPDA
   (ii) a fraction comprising an aqueous phase having a boiling point lower than IPDA;
   (iii) a fraction comprising IPDA
   (iv) a fraction comprising components having a boiling point higher than IPDA; and
IV. subjecting the fraction (ii) to one or more waste-water pretreatment steps, selected from the group consisting of evaporation, oxidation and adsorption.

The production of IPDA by conversion of IPN with ammonia and hydrogen is well-known. IPDA is typically obtained by either (A) converting IPN in the presence of NH3, H2 and a hydrogenation catalyst in a single step or (B) in at least two stages, by first converting IPN fully or partly with NH3 in the presence of an imination catalyst to obtain isophoronenitrileimine (IPNI) and further reacting IPNI with hydrogen in the presence of a hydrogenation catalyst and optionally ammonia.

Preferably IPDA is prepared in two stage process by a) converting IPN with ammonia to IPNI and b) reacting the product from step a) with hydrogen in the presence of a hydrogenation catalyst and ammonia.

### Imination

The first stage (imination) of the two-staged process of converting IPN to IPDA is usually conducted at temperature from 20 to 150°C, preferably 30 to 100°C and more preferably 50 to 90°C and a pressure of 50 to 300 bar, preferably 100 to 250 bar and more preferably 150 to 220 bar. Suitable imination catalysts are acidic oxides, preferably alumina, titania, zirconia and silica. The catalyst loading is preferably in the range of 0.01 to 10, more preferably 0.05 to 7 and even more preferably 0.1 to 5 kg IPN per kg catalyst.

The molar ratio of NH3 to IPN is usually in the range of 5:1 to 500:1, preferably 10:1 to 400:1 and more preferably 20:1 to 300:1.

The imination can be optionally conducted in the presence of a solvent, such as alcohols or ethers, in particularly THF, ethanol or butanol. Most preferably, the imination is not conducted in the presence of a solvent.

The imination can be conducted in one or more pressurized reaction vessels, most preferably the one or more pressurized reaction vessels are one or more tubular reactors where the imination catalyst is arranged in a fixed bed. Preferably the imination is conducted in 1 to 3, more preferably 1 to 2 and even more preferably in one reactor.

The reaction conditions, such as temperature, catalyst, pressure, reactor geometry, are selected in such a manner that the conversion of IPN to INPI is preferably 80% or more, more preferably 90% or more and most preferably 95% or more.

### Hydrogenation

The effluent from the imination step is preferably converted in a second step with hydrogen in the presence of a hydrogenation catalyst and ammonia.

Preferably, the amount of ammonia present during the previous imination step is selected in such a manner, that the ammonia concentration during the hydrogenation step is in a suitable range. A suitable molar ratio of ammonia to IPNI in the hydrogenation step is about 5:1 to 500:1, preferably 10:1 to 400:1 and most preferably 20:1 to 300:1. Additional ammonia can also be optionally added to bring the ammonia concentration into the aforementioned ranges.

The hydrogenation step is conducted in the presence of hydrogen.

The molar ratio between hydrogen and IPNI is preferably in the range of 3:1 to 10000:1, more preferably 4:1 to 5000:1 and most preferably 5:1 to 1000:1.

In a preferred embodiment, hydrogen is added after the imination step. It is however possible, that hydrogen is added prior to the imination step because the imination is usually carried out in the presence of catalysts which do not catalyse the hydrogenation of the imine or nitrile group. The hydrogenation can also be conducted in one or more pressurized reaction vessels.

Most preferably the one or more pressurized reaction vessels are one or more tubular reactors where the hydrogenation catalyst is arranged in a fixed bed. Preferably the hydrogenation is conducted in 1 to 3, more preferably 1 to 2 and even more preferably in one single reactor. The temperature during the hydrogenation is usually in the range of 40 to 200°C , preferably 50 to 150°C, more preferably 60 to 140°C and most preferably 55 to 130°C and a pressure of 50 to 300 bar, preferably 100 to 250 bar and more preferably 150 to 220 bar.

The catalyst load during the hydrogenation is also in the range of 0.01 to 10, preferably 0.05 to 7, more preferably 0.1 to 5 kg IPNI per kg catalyst per hour.

The hydrogenation is preferably carried out in the presence of hydrogenation catalysts, which usually comprise metals or semi-metals from groups 1 to 17 of the Periodic Table as well as the rare earth metals.

Preferred catalyst elements are Ni, Co, Fe, Cu, Ru. Hydrogenation catalysts may also comprise Cr, Cu, Mo. Wo and/or Re.

Preferred hydrogenation catalysts comprise one or more of Ru and Co,
The hydrogenation catalysts can of the so-called Raney-type or the metal-oxide type.

Preferred Raney-type catalysts are Raney-Co-catalysts. The Raney-type catalysts may be supported or unsupported. Suitable Raney-Catalysts are further described in EP1207149, EP 2649042WO2008107226, WO2014086039 and WO2016120235,

The hydrogenation catalysts can also be of the metal-oxide type.
Metal-oxide catalysts are preferably obtained by precipitation of soluble salts of the catalyst elements in the presence of catalyst supports to obtain the corresponding hydroxides, carbonates and oxides and which are usually transformed to the corresponding oxides during a calcination step. The precipitation step may also be conducted without the presence of support materials. Alternatively, the hydrogenation catalyst may be produced by impregnation of a catalyst support with soluble salts of the metals.

The metal-oxides catalysts are usually reduced in the presence of hydrogen prior to their use in the hydrogenation step. The reduced catalysts may be passivated by subjecting the reduced catalysts to an oxygen comprising gas in order to form a passivating and protective oxide layer which allows for safe handling and storage. The passivated catalysts may be reduced or activated prior to their use in the hydrogenation step. Activation and reduction of the metal oxide catalyst is preferably performed in the same reactor, in which the hydrogenation IPNI is performed. The reduction or passivation step may occur prior to the hydrogenation step, but it is also possible to reduce or activate the metal oxide catalysts in-situ during the hydrogenation of IPNI. The unreduced or inactivated catalyst is then transformed into its reduced form by the hydrogen present during the hydrogenation reaction.

Preferred supports are alumina, including but not limited to transitional alumina and non-traditional alumina, titania, zirconia, silica, magnesia, calcium oxide and mixtures thereof.

In a further preferred embodiment, the basicity of the effluent from the imination stage is increased prior or during the subsequent hydrogenation step.

An increase of the basicity can be achieved by the addition of basic compounds or using hydrogenation catalysts which are supported on a basic support. Preferably the basic support comprises elements, such as oxides, of the alkaline metals, preferably Li, Na and K, the alkaline earth metals, preferably Mg and Ca or comprises basic minerals, preferably hydrotalcite, chrysotile or sepiolite.

Preferred basic catalysts are those which are disclosed in WO 2008077852.

In a most preferred embodiment, unsupported hydrogenation catalysts comprising 55 to 98 weight percent of Co, 0.2 to 15 weight percent of P, 0.2 to 15 weight percent of Mn and 0.2 to 15 weight percent of alkali, in particularly Na, are used. Details regarding the specification and production of such catalysts can be found in DE4325847.

Basic compounds can also be added in form of their solutions.

Suitable basic compounds are usually compounds of basic metals, in particularly the oxides, hydroxides or carbonates of alkaline metals, alkaline earth metals or the rare earth metals.

Other suitable basic compounds are ammonium hydroxide and amines.

Preferred basic compounds are oxides, hydroxides and carbonate, in particular Li₂O, Na₂O, K₂O, Rb₂O, Cs₂O, LiOH, NaOH, KOH, RbOH, CsOH, Li₂CO3, Na2CO₃, K₂CO₃, Cs₂CO₃, Rb₂CO₃, MgO, CaO, SrO, BaO, Mg(OH)₂, Ca(OH)₂, Sr(OH)₂, Ba(OH)₂, MgCO₃, CaCO₃, SrCO₃ or
BaCO₃. In particularly preferred basic compounds are LiOH, NaOH and KOH.

The basic compounds are preferably added in form of their solutions in water or other suitable solvents, such as alkanols, like C₁-C₄-alkanols, in particularly methanol or ethanol, or ethers, such as cyclic ether, in particularly THF or dioxane. Preferably the basic compounds are added in form of their aqueous solutions.

The concentration of basic compounds in water or other suitable solvents is usually around 0,01 bis 20 percent by weight, preferably 0, 1 bis 10 percent by weight and more preferably 0,2 bis 5 percent by weight.

The amount of added basic compound is usually determined in such a way as to yield a molar ratio of basic compound to IPNI is in the range of 100: 1 000 000 to 10 000: 1 000 000 and more preferably 200: 1 000 000 to 1000:1 000 000.

Further details regarding the addition of basic compounds prior to the hydrogenation step is disclosed in EP729937 or EP913387, whereas further details regarding the addition of basic compounds during the hydrogenation stage is disclosed in WO 2008077852.

The effluent from the conversion of IPN to IPDA, which is either conducted in a single step or as a two-stage process, comprising imination and hydrogenation as set out above, usually comprises IPDA, hydrogen, ammonia, water, components having a boiling point lower than IPDA and components having a boiling point higher than IPDA.

The effluent from the reductive amination is usually worked-up by first separating hydrogen and ammonia to obtain crude IPDA.

The removal of hydrogen can be effected by flashing the effluent from the IPN-conversion step into a phase separator, such as a flash vessel in order to obtain a gas phase comprising hydrogen and some ammonia and a liquid phase comprising IPDA, ammonia, water and other components.

Ammonia is preferably removed by distillation in one or more ammonia recovery columns.

The effluent from the conversion of IPN with ammonia and hydrogen obtained after the removal of hydrogen and/or ammonia is usually denoted as crude-IPDA.

According to the invention crude-IPDA is separated in one or more steps into
(i) a fraction comprising an organic phase having a boiling point lower than IPDA
(ii) a fraction comprising an aqueous phase having a boiling point lower than IPDA;
(iii) a fraction comprising IPDA; and
(iv) a fraction comprising components having a boiling point higher than IPDA.

Details regarding the separation of crude-IPDA into these components can be found in EP1529028 (BASF), EP3235805 (Evonik), EP3275858 (Evonik) and EP3275858 (Evonik),

In a preferred embodiment, crude-IPDA is introduced into a convention distillation column (Low Boiler Column) to remove fractions (i) and (ii) having a boiling point lower than IPDA at the top of the column. IPDA and components having a boiling point higher than IPDA are removed at the bottom of the Low Boilers Column.

In a preferred embodiment the effluent from the bottom of the Low Boiler Column is separated in one or more steps into a fraction (iii) comprising (IPDA) and a fraction (iv) comprising components having a boiling point higher than IPDA. The separation of IPDA from higher boiling components is usually denoted as fine distillation.

In a preferred embodiment, the distillation of crude IPDA and the fine distillation of IPDA and is conducted in the two-column set-up taught in EP3275858 and EP3235805,

In a more preferred embodiment, the distillation of crude IPDA and the fine distillation of IPDA is conducted in one or more conventional and/or dividing wall columns as taught in EP1529028, whose teaching is also incorporated by reference herein.

In the most preferred embodiments, the distillation of crude IPDA and the fine distillation of IPDA are conducted according to figures 1 to 7 of EP1529028, preferably according to figure 2 or 3 and most preferably according to figure 2 of EP1529028. In these embodiments, fraction (iii) comprising IPDA is obtained as a fraction (iii-a) having a higher cis-trans-ratio (CTR) and a fraction (iii-b) having a lower CTR compared to the CTR of the crude-IPDA. These embodiments have the advantage that the CTR can be adjusted to meet the demands and specifications required in certain down-stream uses and applications of IPDA.

IPDA produced by the process according to the invention can be used as a starting product for preparing isophorone diisocyanate (IPDI), an isocyanate component for polyurethane systems, as an amine component for polyamides or as a hardener for epoxy resins.

As described above, fraction (ii) is usually obtained at the top the Low Boilers Column where it is preferably removed together with fraction (i).

In a preferred embodiment the fractions (i) and (ii) obtained at the top of the Low Boiler Column are fully or almost fully condensed.

The condensate is then preferably fed to a phase separation vessel where the condensate is separated into an aqueous phase and an organic phase. The organic phase comprises fraction (i) and is partly recycled back to the Low Boiler Column as reflux or channeled out of the process.

The aqueous phase comprises fraction (ii), which comprises water and organic impurities, in particularly:
trimethylcyclohexylamine (TMCHA) of formula (I) 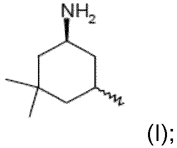
a ring compound (Ring) of formula (II) 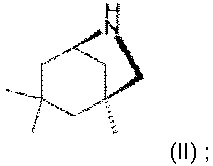 and
aminomethyl-methylcyclohexylamine (AM-MCHA) of formula (III);

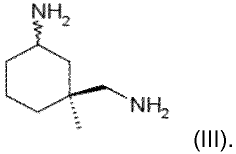

The fraction (ii) usually comprises
0.001 to 10 percent by weight of TMCH,
0.001 to 10 percent by weight of Ring;
0.001 to 10 percent by weight of AM-MCHA; and
70 to 99.997 percent by weight of water.

Preferably fraction (ii) comprises
0.01 to 7 percent by weight of TMCH,
0.01 to 7 percent by weight of Ring;
0.01 to 7 percent by weight of AM-MCHA; and
79 to 99.97 percent by weight of water.

More preferably fraction (ii) comprises
0.015 to 3 percent by weight of TMCH,
0.1 to 5 percent by weight of Ring;
0.1 to 5 percent by weight of AM-MCHA; and
87 to 99.785 percent by weight of water.

In a more preferred embodiment, fraction (ii) comprises low levels of HCN or nitrile compounds, in particularly isophorone nitrile amine (IPNA). Preferably fraction (ii) comprises 0.01 weigh percent or less, more preferably 0.005 weight percent or less and most preferably 0.001 weight percent or less of HCN and nitrile compounds. Nitrile compounds are organic compounds comprising one or more nitrile groups.

According to the invention, fraction (ii) is subjected to wastewater treatment (step IV) which comprises one or more pretreatment steps A) selected from evaporation, oxidation and adsorption.

### Evaporation

In a preferred embodiment, one of the one or more pretreatment steps is an evaporation step. In the evaporation step, organic impurities comprised in fraction (ii) are transferred from the liquid phase to the vapor phase.

The evaporation step is preferably conducted in a device which creates a large surface area of the liquid phase to improve the transfer of organic impurities from the gas to the liquid phase.

### Stripping

In a more preferred embodiment, the evaporation step is a stripping step.

The stripping step is preferably conducted in a stripping device.

The stripping device can be a tray or plate stripping column, a packed column, a spray tower, a bubble column, a membrane contactor, a radial flow scrubber, a jet scrubber, a Venturi scrubber or a rotary spray scrubber.

Preferably, the stripping device is a tray stripping column or a packed stripping column.

An outlet for treated water is usually positioned at the bottom of the stripping column

An outlet for the stripping gas, comprising stripped out organic impurities, is usually positioned at the top of the stripping column.

The stripping column usually comprises a stripping zone.

The stripping zone of the stripping column is usually situated between the sump of the column and the top of the column and denotes the area in which the fraction (ii) is contacted with the stripping gas.

The stripping zone usually comprise internals to improve the contact between fraction (ii) and the stripping gas and to increase the surface area of liquid fraction (ii) In order to improve enhance the mass transfer from the liquid phase to the gas phase.

The internals are usually trays or packings.

In a trayed stripping column, one or more trays are arranged along the vertical axis of the column to enhance mass transfer from the liquid to the vapor phase.

Tray types include sieve, valve, Thormann trays and bubble cap trays. Preferred trays are sieve trays.

Packed stripping columns can be filled with a random, dumped packing (random-packed column) or with structured packings, which are arranged or stacked in the column (stack packed column).

Columns having random packings can be filled with different shaped bodies. Heat and mass transfer are improved by the increase in the surface area caused by the shaped bodies, which are usually about 25 to 80 mm in size. Known examples are the Raschig ring (a hollow cylinder), Pall ring, Hiflow ring, Intalox saddle and the like. The random packings can be introduced into the column in an ordered manner, or else randomly (as a bed). Possible materials include glass, ceramic, metal and plastics.

Structured packings are a further development of ordered random packings. They have a regular structure. As a result, it is possible in the case of packings to reduce pressure drops in the gas flow. There are various designs of structured packings, for example woven packings or sheet metal packings. Materials used may be metal, plastic, glass and ceramic.

In a preferred embodiment, the stripping column is equipped with a random packing of Pall rings The stripping zone can be subdivided in different sections, preferably into 1 to 4 sections. Between the sections, support trays and distribution trays can be arranged. Support trays usually support the packing to hold it in place. Distribution trays usually help to distribute the liquid even over the cross section of the column.

The total number of theoretical plates in the stripping section is usually between 2 and 50, preferably 3 and 40 and more preferably 5 and 20.

If the stripping zone comprises random or structured packings, the height of the stripping zone is typically in the range of 0.5 m to 10 m, preferably 1 m to 5 m and more preferably 1.5 to 4 m. Fraction (ii) is usually fed in the upper area of the column, preferably above or at the top of the stripping zone.

The feed temperature at the inlet to the stripper is preferably 70°C or more, preferably 80°C or more and more preferably 90°C or more. Fraction (ii), which usually has a temperature of around 20 to 60°C, preferably 20 to 50°C and more preferably 35 to 45°C coming from the phase separator in which fractions (i) and (ii) are separated, is preferably heated to the above-mentioned inlet temperatures. Heating of a feed stream is usually conducted using heat exchangers. Preferably, the feed stream is directly or indirectly heated with the pre-treated water drawn-off from the sump of the stripper as further described below.

The zone below the stripping zone is usually referred to as the column sump. In this zone, the pretreated water is usually collected and drawn-off via an outlet which is usually positioned at the bottom of the column.

According to a preferred embodiment of the invention, at least a portion of the pretreated water drawn-off from the bottom of the stripper is fed to the additional wastewater treatment step B). In a preferred embodiment, at least a portion of the pretreated water drawn-off from the bottom of the stripper is used to preheat the feed comprising fraction (ii).

Preferably, preheating occurs by recycling at least a portion of the pretreated water drawn-off from the bottom of the stripper to the feed of the stripping column and by directly contacting or mixing the recycling stream with the feed stream. Mixing the cooler feed stream comprising fraction (ii) with the warmer recycling stream from the sump of the stripping column results in a feed stream having a higher feed temperature. Preferably, around 1/5 to 4/5 (w/w), more preferably about 1,5/5 to 3/5 (w/w) and most preferably about 2/5 (w/w) of the pretreated water drawn-off from the bottom of the stripping column is recycled to the feed stream. The ratio of the cooler feed stream comprising fraction (ii) to the warmer recycling stream from the bottom of the stripping column is preferably 1:4 to 10:2 (w/w), more preferably 2:4 to 6:2 (w/w) and most preferably 2:2 to 4:2 (w/w).

In another preferred embodiment, the pretreated water drawn-off from the bottom of the stripper is used to heat the feed stream comprising fraction (ii) by indirectly contacting the feed stream with at least a part of the stream from the sump of the stripper. Indirect contacting is preferably conducted in a heat-exchanger, preferably a plate heat exchanger, a spiral heat exchanger, a shell and tube heat exchangers or a plate and shell heat exchanger.

In a most preferred embodiment, the pretreated water drawn-off from the sump of the stripping column is split into a recycling stream, which is brought into direct contact with the feed stream, and an effluent stream, which is used to indirectly heat the feed stream via a heat exchanger. The weight ratio of the recycling stream to the effluent stream is preferably 1:9 to 8:9, preferably 2:9 to 7:9 and most preferably 3:9 to 6:9.

The above-mentioned embodiments have the advantageous, that the content of Ring in the pre-treated water from the sump of the stripper can be further reduced.

In a further preferred embodiment, a part of the pretreated water obtained in the sump of the stripper is introduced into a reboiler. In the reboiler, at least a part of the pretreated water is transformed into steam. Suitable reboilers are usually kettle reboilers, reboilers of the thermosiphon type (natural circulation reboilers) or forced circulation reboilers. The reboiler is usually situated outside the stripping column and connected to the outlet, where the pretreated water is drawn-off.

The reboiler is usually operated at a temperature suitable to transform the treated water into steam at the given pressure, and is preferably in the range of 100° to 150°C, more preferably 100 to 140°C and more preferably 100 to 130°C. The generated steam is usually reintroduced back into the stripping column below the stripping section, preferably into the sump of the regenerator. The amount of steam generated in the reboiler and introduced into the stripping column depends on the size of the column. Preferably the weight ratio of steam to the feed stream at the inlet of the stripper is in the range of 1:50 to 25:50, more preferably 1:30 to 20:30 and most preferably 1:15 to 3:15

The stripper is preferably operated at a pressure of 0.1 to 5, preferably 0.5 to 3 and more preferably 0.8 to 1.5 bar. Most preferably the stripper is operated at ambient atmospheric pressure. The steam is preferably introduced via a gas distribution device which helps to evenly distribute the steam across the cross-section of the stripping column, such as a distribution ring, a sinter plate, a perforated plate or a nozzle.

In another preferred embodiment, the stripping column is lacking a reboiler. The steam required for stripping is then generated in an external evaporator. The evaporator is preferably a steam generator. Steam generation in integrated chemical plants is usually centrally provided and the generated steam can be introduced into the stripping column via pipelines from a steam network. The steam is preferably introduced into the stripping column in the same manner and in the same way in which steam regenerated in an internal reboiler would be introduced into the stripping column.

In a further preferred embodiment, the stripping gas is not steam, but another stripping gas, which is inert against water, and which is preferably nitrogen or air. Other stripping gases can also be used, such as noble gases, like He, Ne and Ar, but such gases are usually not chosen due to economic reasons. Preferably air and nitrogen are used as stripping gases due to their ready availability and lower costs. The stripping gas is preferably introduced in the same manner as an external steam stream.

The top of the stripping column is usually connected with a condenser.

Suitable condensers are condensers with a cooling coil or plate heat exchangers, field tube heat exchangers or tube bundle heat exchangers.

The condenser is usually operated with a cooling medium, preferably water, having a temperature of 10 to 40°C, preferably 15 to 35°C and more preferably 20 to 30°C at the inlet of the condenser.

If steam is used as a stripping gas, a condensate comprising condensed steam and organic impurities is obtained. Uncondensed parts of the gaseous stream leaving the top of the column are preferably send to an incinerator for combustion.

The condensate, comprising water and organic impurities, is preferably fed to phase separator, where it is divided into an organic phase, comprising the undesired impurities, and an aqueous phase.

The aqueous phase obtained at the top of the stripper may be directly fed to the treatment step B).

In a preferred embodiment, the aqueous phase obtained at the top of the stripper is recycled to the IPDA-process, preferably step III and more preferably to the Low Boiler Column where fractions (i) and (ii) are removed. In particularly the aqueous phase obtained at the top of the stripper is recycled to the phase separator of the Low Boiler Column or to the feed of the Low Boiler Column. Recycling of the aqueous phase to the IPDA-process has the advantage that the infrastructure of the IPDA-plant can be used to handle the wastewater streams since they the streams can be combined with the wastewater already generated during IPDA production.

The organic phase obtained in the phase separator at the top of the stripper can be channeled out of the process and sent to an incinerator or flare for combustion.

In a preferred embodiment, the organic phase is recycled to the IPDA-process, preferably to step III and more preferably into the Low Boiler Column, in particularly preferably the phase separator of the Low Boiler Column or the feed of the Low Boiler Column.

Recycling of the organic phase to the IPDA-process has the advantage that the concentration of organics in the phase separator of the Low Boiler Column is increased, enabling a more efficient phase separation in the phase separator of the Low Boiler Column. In addition, the organic phase can be combined with the organic phase obtained in the Low Boiler Column and can be used as reflux to improve the separation of low boilers and IPDA in the Low Boiler Column. In addition, the infrastructure of the IPDA-process can be used to further handle the organic fraction, e.g. existing flares and incinerators can be used to dispose of at least a portion of the organic impurities obtained in the stripping step.

In a preferred embodiment, both the aqueous phase and the organic phase obtained in the phase separator of the stripper are recycled to the IPDA-process, as described above. The phases can be recycled separately, or they can be re-combined before recycling.

If a stripping gas other than steam is used, the condensate obtained at the top of the stripping column usually comprises organic impurities and some stripped out water.

The condensate is preferably recycled back to the phase separator of the Low Boiler Column.

In another embodiment, the condensate is recycled back to the feed of the Low Boiler Column. In still another embodiment the condensate is directly send to an incinerator.

In a preferred embodiment, the condensate obtained at the top of the stripper is also sent to a phase separator and divided into an aqueous phase and an organic phase, comprising the organic impurities. As set out above, the phases from the phase separator of the stripper can be recycled jointly or separately recycled back to the phase separator of the Low Boiler Column. The pretreated water from the sump of the stripper is depleted in TMCHA of formula (i), Ring of formula (ii) and AM-MCHA of formula (iii).

Preferably the concentration of each of the three components is 0.1 weight percent or less, more preferably 0.075 weight percent or less and most preferably 0.05 weight percent or less. When using a stripper in the pretreatment step (A), especially the Ring of formula (ii) can be reduced by a factor or 10 to 80, more preferably 20 to 70 and most preferably 40 to 60.

### Adsorption

In a preferred embodiment, the pretreatment step A) is an adsorption step. Further details regarding the treatment of water by adsorption can be found in Bart, H.-J. and von Gemmingen, U. (2005). Adsorption. In Ullmann's Encyclopedia of Industrial Chemistry, (Ed.). doi:10.1002/14356007.b03_09.pub2 and Worch, E. (2015). Water, 2. Treatment by Adsorption Processes. In Ullmann's Encyclopedia of Industrial Chemistry, (Ed.). doi:10.1002/14356007.o28_o13.pub2,

The contacting of the adsorbent with fraction (ii) is preferably carried out in a slurry or fixed bed reactor.

In principle, slurry adsorbers can be operated either discontinuously as batch adsorbers or continuously as continuous-flow slurry adsorbers.

In batch adsorbers, the adsorbent is usually in contact with the adsorbate solution until the equilibrium is reached.

The adsorbent can usually be separated from the pretreated water by sedimentation, filtration or centrifugation.

In a preferred embodiment, the contact of fraction (ii) with the absorbent is carried out in a fixed bed reactor. The preferred direction of flow is usually from top to bottom. If the resistance of the bed becomes too high, the bed may be cleaned by purging and loosening of the layer. Preferably, the fixed bed adsorbers are designed as closed pressure filters or as open gravity filters with circular or rectangular cross section. The fixed bed reactors are typically made of corrosion-resistant steel (stainless steel or steel coated with polymers) or concrete. The adsorbent in a fixed bed adsorber is located on a perforated bottom, and the water usually streams downward through the adsorbent bed.

Typical adsorbents are activated carbon, silica, silica gel, activated alumina, clay minerals and pillared clays, polymeric resins and isopolyacids of iron, aluminum or silicon, preferred adsorbents are activated alumina, polymeric resins and activated carbon.

In a preferred embodiment activated alumina is used as an adsorbent. Activated alumina is preferably used in the form of gamma-alumina with a specific surface area of 200 to 400, preferably 300 to 350 m²/g, a pore volume of 0.3 to 0.6, preferable 0.4 to 0.5 cm³/g and a bulk density of 600 to 900, preferably 700 to 800 g/L.

In a further preferred embodiment adsorption resins are used as adsorbents. Adsorber resins are preferably resins having a specific surface area of 300 to 2000, preferably 400 to 1500 m²/g, a porosity of 30 to 70, preferably 35 to 65 volume percent and a bulk density of 500 to 800, preferably 650 to 750 g/L. Such resins are commercially available and are usually obtained by polymerization of styrene, divinylbenzene and/or acrylic acid. Adsorber resins are normally regenerated by extraction with methanol, acetone, toluene and other solvents. Appropriate processing of the extracts permits the solvents to be recovered and the eliminated organic materials to be isolated.

In the most preferred embodiment activated carbon is used as an adsorbent. Activated carbon is preferably used in granular or powder form and preferably has a specific surface area of 300 to 4000, preferably 500 to 1000 m²/g, a pore volume of 0.1 to 1.0, preferably 0.3 to 0.8 cm³/g and a bulk density of 100 to 700, preferably 300 to 550 g/L. Activated carbon can be produced from different carbon-containing raw materials, such as wood, wood charcoal, peat, lignite and lignite coke, hard coal and coke, bituminous coal, petrol coke as well as residual materials such as coconut shells, sawdust or plastic residuals.

In a most preferred embodiment, the adsorbent is activated carbon in granular form (granular activated carbon = GAC), and contacting is carried out in a fixed bed reactor.

The GAC-fixed bed reactor is preferably operated as follows:
Bed height: 1 to 10, preferably 1.5 to 7.5 and more preferably 2 to 5 m;
Cross sectional area: 1 to 50, preferably 2.5 to 40 and more preferably 5 to 30 m²;
Velocity: 1 to 50, preferably 2.5 to 30 and more preferably 5 to 20 m/h;
Effective contact time: 1 to 30, preferably 1.5 to 20 and more preferably 2 to 10 minutes;
GAC particle diameter: 0.1 to 10, preferably 0.2 to 7.5 and more preferably 0.5 to 5 mm;
Bed volume: 1 to 100, preferably 5 to 75 and more preferably 10 to 50 m³;
Bed porosity: 0.1 to 1, preferably 0.2 to 0.75 and more preferably 0.35 to 0.45.

The exact details of the fixed bed reactor can be designed on the basis of an appropriate breakthrough curve model or an appropriate scale up procedure, such as the rapid small-scale column test.

Until the breakthrough, the water can be continuously treated, but if a breakthrough occurs, the activated carbon usually needs to be reactivated. Activated carbon can be reactivated by a thermal process by gas activation. To overcome the problem of activation, multiple adsorbers are typically used in the adsorption process. The different adsorbers can be connected in series or in parallel. Preferably, at least two adsorbers or more, more preferably three adsorbers or more and most preferably four adsorbers or more are used, with one adsorber usually being disconnected from the other adsorbers to allow regeneration of the adsorbent in the disconnected adsorber.

If the pretreatment step (A) is an absorption step, the component AM-MCHA of formula (iii) can be reduced by 98 weight percent or more, preferably 98.5 weight percent or more, more preferably 99 weight percent or more and most preferably 99.5 weight percent or more.

### Oxidation

In a further preferred embodiment, fraction (ii) can also be pretreated using an oxidation step. The oxidation step is characterized therein that fraction (ii) is brought into contact with an oxidant. Details regarding the oxidative treatment of water can be found in Lutze, H.V. (2016). Water, 6. Treatment by Oxidation Processes, in Ullmann's Encyclopedia of Industrial Chemistry, (Ed.). doi:10.1002/14356007.o28_o17.pub2 or Simmler, W. (2011). Wastewater, 4. Chemical Treatment, in Ullmann's Encyclopedia of Industrial Chemistry, (Ed.). doi:10.1002/14356007.o28_o10.

The oxidant is preferably a substance which is able to oxidize organic impurities, in particular AMCHA, which are less degradable in a biological wastewater treatment to degradation products which are more easily degradable in a biological wastewater treatment step,

Preferably, the oxidant is hydrogen peroxide, activated hydrogen peroxide (e.g. activated by iron (such as the Fentox-process), ultra-violet light or ozone), peroxymonosulfates, such as Caroat or Oxone, peroxdisulfuric acid, peroxymonosulfuric acid, hypochlorite, chlorite, chlorate, perchlorate and other analogous halogen compounds, permanganate and perborates. Oxidation can also be carried out by radiation with UV-light, by the introduction of oxygen at higher temperatures, such as the hydrothermal treatment of water with oxygen as oxidizer, also known as wet oxidation) or the supercritical oxidation of water, where oxidation occurs with oxygen at pressures and temperatures where water becomes a fluid.

In a preferred embodiment, the oxidant is hydrogen peroxide or activated hydrogen peroxide. Hydrogen peroxide is preferably used in the form of an aqueous solution between 10 to 50 percent (w/v), more preferably 20 to 40 percent (w/v) and even more preferably 30 percent (w/v). Oxidation can be conducted in a broad pH-range, preferably in a pH-range of 5 to 11, more preferably 6 to 10. Oxidation is preferably conducted in a temperature range of 10 to 50°C, more preferably 15 to 45°C and even more preferably 20 to 40°C.

Oxidation is preferably conducted in a tank, a stirred tank or continuously in a cascade of stirred tank reactors or a plug flow reactor, such as a tubular plug flow reactor.

The contact time between the oxidant and the water to be treated is usually in the range of 5 minutes or more, preferably 10 minutes or more, more preferably 15 minutes or more and most preferably 30 minutes or more.

More preferably, the contact time between water and the oxidant is usually in the range of 5 to 360 minutes, preferably 10 to 240 minutes and more preferably 30 to 90 minutes.

The amount of added oxidant to organic impurities is preferably in the range of 10:1 to 200:1, more preferably 20:1 to 150:1, even more preferably 30:1 to 125:1 and most preferably 40:1 to 100:1.

Oxidation can also occur using photocatalysts, such as titania. Photocatalysis may be enhanced by addition of co-oxidants, such as hydrogen peroxide or oxygen, and/or by intensifying the light intensity, e.g. by radiation with UV-light or lamps.

In a preferred embodiment, one of the one or more pretreatment steps is a stripping step as described above. Stripping has the advantage that it can be easily integrated into the IPDA production process. The separated organic impurities and the pre-treated water can be recycled to different parts of the IPDA-process, in particular the phase separator of the Low Boiler Column or the feed of the Low Boiler Column. It is therefore possible to utilize existing infrastructure for the further handling and disposure of the separated impurities and water. The separated impurities can be either used as reflux to improve the separation of light boilers from IPDA or they can be obtained in a more concentrated form to allow more efficient disposal, e.g. by incineration and/or thermic recycling. If stripping is conducted with steam as a stripping gas, it is further possible to use the steam infrastructure of an integrated chemical site. In this way, a stripping gas (steam) can be utilized which is readily available and which has a good separation effect. The efficient removal of organic impurities by stripping reduces the organic load to the subsequent organic wastewater treatment plant. A stripping step is particularly useful in depleting the so-called Ring component of formula (ii).

In a further preferred embodiment, one of the one or more pretreatment steps is an oxidation step. It has been found that some organic impurities, in particularly AM-MCHA of formula (III) is not easily removed in a stripping step. If fraction (ii) comprises non-negligible amounts of AM-CHA, a degradation of AM-CHA may therefore be achieved by an oxidation pretreatment.

In yet another preferred embodiment, one of the one or more pretreatment steps is an adsorption step. Adsorption is an alternative to oxidation for such impurities, which are not easily removable by a stripping pretreatment. In particularly, adsorption has been found to be an efficient method to remove AM-MCHA.

In a very particularly preferred embodiment, pre-treatment of fraction (ii) is effected by conducting more than one pretreatment steps.

A most preferred embodiment is the combination of a stripping pretreatment followed by either an adsorption pretreatment or an oxidation pretreatment. This combination combines the advantages of the stripping step while also allowing efficient removal of those organic impurities, which are not easily removable by stripping. This combination is therefore particularly useful if non-negligible amounts of AM-MCHA is present in fraction (ii).

All pretreatment steps have the advantage that the organic load to the biological wastewater treatment is reduced, thereby reducing its oxygen demand and therefore lowering the COD values.

### Biological Wastewater Treatment Step

According a preferred embodiment of the present invention, the pretreated water from IPDA production is subsequently fed to a biological wastewater treatment step B).

Accordingly, the present invention is also directed to a method for the production of IPDA comprising the steps of:
I. converting IPN with ammonia and hydrogen to IPDA,
II. removing hydrogen and/or ammonia from the effluent from step I
III. separating the effluent from step II in one or more steps into
   (i) a fraction comprising an organic phase having a boiling point lower than IPDA
   (ii) a fraction comprising an aqueous phase having a boing point lower than IPDA;
   (iii) a fraction comprising IPDA
   (iv) a fraction comprising components having a boiling point higher than IPDA; and
IV. subjecting the fraction (ii) to
   A) one or more waste-water pretreatment steps, selected from the group consisting of evaporation, oxidation and adsorption; and
   B) a biological waste-water treatment step.

According to this embodiment, fraction (ii) is subjected to one or more pretreatment steps, selected from the group consisting of evaporation, oxidation and absorption as previously described.

Biological wastewater treatment for industrial wastewater is described in detail in Ullmann's Encyclopedia ("Wastewater, 1. Introduction" (Horn, H., Bennemann, H., Blöcher, C., Geißen, S.-U., Marzinkowski, J.M. and Scherer, U. (2017), "Wastewater", 1. Introduction, in Ullmann's Encyclopedia of Industrial Chemistry, (Ed.). doi:10.1002/14356007.b08 001.pub3) and "Wasterwater, 2. Aerobic Biological Treatment", Telgmann, L., Horn, H., Schönberger, H., Gescher, J., Abbt-Braun, G., Blöcher, C., Gisselmann, J., Blesgen, A., Wagner, M., West, S., Mann, T., Berger, M., Kern, G., Lemke, J., Klockner, D., Neuber, E., Hebbel, G.-W., Werthmann, U., Klinsmann, G., Lawson, J.F., Meyer, H.G., Müller, M., Balser, K., Maier, W., Frieser, J., Thüer, M. and Ma-laszkiewicz, J. (2020), "Wastewater, 2. Aerobic Biological Treatment", in Ullmann's Encyclopedia of Industrial Chemistry. doi:10.1002/14356007.o28_o08.pub2).

Preferably, the biological wastewater treatment is carried out according to the process scheme described in Figure 4.8-2 in Völker, E., Zimmer, G. and Elendt-Schneider, B. (2010) "Verantwor-tungsvoller Umgang mit Wasser am Beispiel der BASF SE Ludwigshafen", in Warnsignal Klima: Genug Wasser für alle? (eds. J.L. Lozán, H. Graßl, L. Karbe, et al.), Climate Service Center Germany, pp. 503-511.

Surprisingly it has been found that the aqueous phase obtained during the removal of components having a boiling point lower than the boiling point of IPDA comprises different organic species, such as TMCHA of formula (I), a Ring of formula (II) and AM-MCHA of formula (III), even after the separation of the organic phase in a phase separator of the Low Boiler Column. It has further been found that these organic species are only slightly biodegradable in a biological wastewater treatment step leading to an increase in the COD value or an increase of organic impurities in the treated water which is disposed to the environment. It has further been found that at least some of the impurities can be efficiently removed by selected pretreatment steps. Evaporation, in particularly stripping, has been found to be efficient in the removal of TMCHA and the Ring, whereas the amount of AM-MCHA can be reduced by a pretreatment based on adsorption or oxidation. It was found that stripping can be readily integrated into an IPDA production process allowing the recycling and concentration of separated streams and the utilization of already existing infrastructure to reduce investment costs. It has further been found that the combination of pretreatment by stripping and pretreatment by adsorption or the combination of pretreatment by stripping and pretreatment by oxidation are particularly effective in removing impurities produced in IPDA manufacturing and to lower the chemical oxygen demand in subsequent step B).

The benefits of the invention are demonstrated by the following examples:

### Example 1: Pre-treatment in a stripper

The examples are based on calculations performed using a process simulation model.

The simulations have been performed using CHEMASIM^{®}. For the calculation of thermodynamic properties of pure components, like the vapor pressure, DIPPR correlations have been used. For the description of phase equilibria, the ideal gas law is used to describe the vapor phase and the NRTL excess Gibbs energy model is used for the description of the liquid phase. The parameters of the DIPPR correlations and the parameters of the NRTL model were adjusted to experimental data. For the components, for which no experimental data are available, the UNIFAC group contribution method was used for the description of have been adjusted to reproduce experimental and plant data with very good accuracy. the liquid phase in phase equilibria calculations. The distillation columns have been modelled and calculated using the equilibrium stage model. The employed simulation and thermodynamic property models have been adjusted to reproduce experimental and plant data with very good accuracy.

A feed stream (fraction (ii) from the phase separator used to separate organic and aqueous low boilers) having a temperature of 40°C was heated in a heat exchanger to about 90°C. The heat medium for the heat exchanger was the pre-treated water drawn-off from sump from the stripping column. The heated feed stream (300 kg/h) was fed to the top of a stripping column above the stripping section. 37 kg/h of steam having a temperature of 100°C was introduced into the bottom of the stripping column below the stripping section. A gas stream of 21 kg/h was drawn-off at the top of the stripping column and condensed in a condenser. 516 kg/h of pre-treated water were drawn-off from the bottom of the stripping column. Of the 516 kg/h, 200 kg/h were recycled and mixed with the heated feed stream prior to entering the stripping column. 316 kg/h of the pre-treated water were sent to a biological waste-water treatment step.

The composition of the streams is given in Table 1 below.

**Table 1: Composition (w/w) of the stream in the stripping pre-treatment step**

| | feed stream(cold) | Pre-treated water from the sump of the stripping column | Recycling stream from sump of stripping column to feed stream | Gas phase removed at the top of the stripping column | Pre-treated water sent to organic waste-water treatment step |
|---|---|---|---|---|---|
| Water | 0,9804 | 0,9974 | 0,9974 | 0,7827 | 0,9974 |
| TMCHA | 0,00176 | 0,000169 | 0,000169 | 0,0205 | 0,000169 |
| Ring | 0,01557 | 0,000298 | 0,000298 | 0,197 | 0,000298 |
| AM-MCHA | 0,00194 | 0,00186 | 0,00186 | traces | 0,001886 |

The Ring component of formula (ii) can be drastically reduced from 1,557 weight percent to 0,0298 weight% in the pre-treated water sent to the biological waste-water treatment step.

### Example 2: Pre-treatment by absorption

Carbon black of the type "Chemviron Carbon C201" was used.

Carbon black in a amount set out in Table 2 was mixed in 100 ml flasks with 100 ml of fraction (ii) comprising a concentration of 150 mg/l of AM-MCHA of formula (iii). The flasks were shaken 24 hours at 300 rev/min on a laboratory shaker (type: IKA KS 250).

After shaking, the content of the flask was filtered to remove the carbon black and the concentration of AM-MCHA was determined (see Table 2).

**Table 2:**

| | Carbon black per flask (mg/100 ml) | Carbon black per flask (mg/l) | Rest concentration of AM-MCHA [mg/l] | Carbon black loading [mg AM-MCHA per g active carbon] | Percentual elimination of AM-MCHA by adsoprtion [%] |
|---|---|---|---|---|---|
| 1 | 0 | 0 | 150,0 | 0 | 0 |
| 2 | 100 | 1000 | 77,0 | 73 | 48,7 |
| 3 | 500 | 5000 | 2,2 | 30 | 98,5 |
| 4 | 1000 | 10000 | 0,3 | 15 | 99,8 |
| 5 | 2000 | 20000 | 0,1 | 7 | 99,9 |

At a loading of 30 mg AM-MCHA per g active carbon, the amount of AM-MCHA in the pre-treted water sent to an organic waste-water treatment plant can be reduced by more than 98%.

### Example 3: Pre-treatment by oxidative treatment

Fraction (ii) was treated with different amounts of hydrogen peroxide.

Afterwards, the concentration of residual AM-MCHA was determined.

The residual AM-MCHA concentration was plotted against the amount of added hydrogen peroxide. A linear regression of the data was performed (y=2,8609+574,84).

According to the above formula, it was determined that 1 g of a 30% solution of hydrogen peroxide or 0.3 g of pure hydrogen peroxide were able to eliminate 18 mg/l of AM-MCHA.

From the examples it can be inferred that a pre-treatment in a stripping column is particularly effective in removing the Ring component of formula (ii), whereas adsorption and oxidative treatment is particularly in removing the component AM-MCHA of formula (iii).

Both components can be eliminated if either stripping and adsoprtion or stripping and oxidative treatment are combined, prior to feed the waste-water to a biological waste-water treatment step.

## Claims

1. A method for the production of IPDA comprising the steps of:
I. converting IPN with ammonia and hydrogen to IPDA,
II. removing hydrogen and/or ammonia from the effluent from step I
III. separating the effluent from step II in one or more steps into
(i) a fraction comprising an organic phase having a boiling point lower than IPDA
(ii) a fraction comprising an aqueous phase having a boing point lower than IPDA;
(iii) a fraction comprising IPDA
(iv) a fraction comprising components having a boiling point higher than IPDA; and
IV. subjecting the fraction (ii) to one or more waste-water pretreatment steps, selected from the group consisting of evaporation, oxidation and adsorption.

2. A method according to claim 1, wherein one of the one or more pretreatment steps IV is a stripping pretreatment step.

3. A method according to at least one of claims 1 to 2, wherein the stripping pretreatment step is carried out in a stripping column and with steam as a stripping gas.

4. A method according to at least one of claims 1 to 3, in which the condensate obtained at the top of the stripping column is separated into an organic phase and an aqueous phase.

5. A method according to at least one of claims 1 to 4, wherein the organic phase and/or the aqueous phase are recycled to a phase separator used to separate fractions (i) and (ii) after the removal of components having a boiling point lower than IPDA from the effluent from step II.

6. A method according to at least one of claims 3 to 5, wherein at least a part of the pre-treated water obtained at the bottom of the stripping column is used to directly or indirectly heat the feed of fraction (ii) to the stripping column.

7. A method according to at least one of claims 1 to 6, wherein the steam used as a stripping gas is (i) generated in a reboiler connected to the sump of the stripping column or (ii)is provided from a steam networks of an integrated chemical plant.

8. A method according to at least one claims 1 to 7, wherein one of the one or more pretreatment steps IV is an oxidation pretreatment step.

9. A method according to claim 8, wherein the molar ratio of added oxidant to organic impurity is in the range of 10:1 to 100:1.

10. A method according to at least one of claims 8 to 9, wherein the chemical oxidant is hydrogen peroxide.

11. A method according to at least one claims 1 to 10, wherein one of the one or more pretreatment steps IV is an adsorption pretreatment step.

12. A method according to claim 11, wherein the adsorbent is activated carbon.

13. A method according to at least one of claims 1 to 13, wherein fraction (ii) comprises
0.001 to 10 percent by weight of trimethylcyclohexylamine (TMCHA) of formula (I) 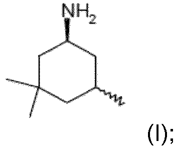
0.001 to 10 percent by weight of a ring compound (Ring) of formula (II) 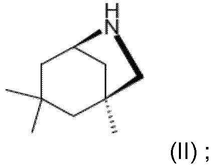
0.001 to 10 percent by weight of aminomethyl-methylcyclohexylamine (AM-MCHA) of formula (III); 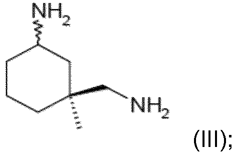 and
70 to 99.997 percent by weight of water.

14. A method according to at least one of claims 1 to 13, wherein fraction (ii) comprises less than 0.01 percent by weight of HCN or nitrile compounds.

15. A method according to at least one of claims 1 to 14, wherein the pretreatment step is a combination of a stripping pretreatment step and an adsorption pretreatment step or a combination of a stripping pretreatment step and an oxidation pretreatment step.

16. A method according to claim 1, wherein fraction (ii) is subjected to a biological waste-treatment step after being subject to one or more waste-water pretreatment steps, selected from the group consisting of evaporation, oxidation and adsorption.

## Patentansprüche

1. Verfahren zur Herstellung von IPDA, umfassend die Schritte:
I. Umwandeln von IPN mit Ammoniak und Wasserstoff zu IPDA,
II. Entfernen von Wasserstoff und/oder Ammoniak aus dem Abstrom aus Schritt I
III. Trennen des Abstroms aus Schritt II in einem oder mehreren Schritten in
(i) eine Fraktion umfassend eine organische Phase mit einem tieferen Siedepunkt als IPDA
(ii) eine Fraktion umfassend eine wässrige Phase mit einem tieferen Siedepunkt als IPDA;
(iii) eine Fraktion umfassend IPDA
(iv) eine Fraktion umfassend Komponenten mit einem höheren Siedepunkt als IPDA, und
IV. Unterwerfen der Fraktion (ii) einem oder mehreren Abwasser-Vorbehandlungsschritte ausgewählt aus der Gruppe bestehend aus Verdampfung, Oxidation und Adsorption.

2. Verfahren nach Anspruch 1, wobei einer des einen oder der mehreren Vorbehandlungsschritte IV ein Stripping-Vorbehandlungsschritt ist.

3. Verfahren nach wenigstens einem der Ansprüche 1 bis 2, wobei der Stripping-Vorbehandlungsschritt in einer Strippkolonne und mit Dampf als Strippgas durchgeführt wird.

4. Verfahren nach wenigstens einem der Ansprüche 1 bis 3, wobei das an dem Kopf der Strippkolonne erhaltene Kondensat in eine organische Phase und eine wässrige Phase aufgetrennt wird.

5. Verfahren nach wenigstens einem der Ansprüche 1 bis 4, wobei die organische Phase und/oder die wässrige Phase nach dem Entfernen von Komponenten mit einem tieferen Siedepunkt als IPDA aus dem Abstrom aus Schritt II in einen Phasentrenner zurückgeführt werden, der zur Trennung der Fraktionen (i) und (ii) verwendet wird.

6. Verfahren nach wenigstens einem der Ansprüche 3 bis 5, wobei wenigstens ein Teil des an dem Sumpf der Strippkolonne erhaltenen vorbehandelten Wassers verwendet wird, um den Zulauf der Fraktion (ii) zur Strippkolonne direkt oder indirekt zu erhitzen.

7. Verfahren nach wenigstens einem der Ansprüche 1 bis 6, wobei der als Strippgas verwendete Dampf (i) in einem mit dem Sumpf der Strippkolonne verbundenen Reboiler erzeugt wird oder (ii) aus einem Dampfnetz einer integrierten chemischen Anlage bereitgestellt wird.

8. Verfahren nach wenigstens einem der Ansprüche 1 bis 7, wobei einer des einen oder der mehreren Vorbehandlungsschritte IV ein Oxidations-Vorbehandlungsschritt ist.

9. Verfahren nach Anspruch 8, wobei das Molverhältnis von zugegebenem Oxidationsmittel zu organischer Verunreinigung in dem Bereich von 10:1 bis 100:1 liegt.

10. Verfahren nach wenigstens einem der Ansprüche 8 bis 9, wobei das chemische Oxidationsmittel Wasserstoffperoxid ist.

11. Verfahren nach wenigstens einem der Ansprüche 1 bis 10, wobei einer des einen oder der mehreren Vorbehandlungsschritte IV ein Adsorptions-Vorbehandlungsschritt ist.

12. Verfahren nach Anspruch 11, wobei das Adsorptionsmittel Aktivkohle ist.

13. Verfahren nach wenigstens einem der Ansprüche 1 bis 13, wobei Fraktion (ii) umfasst
0,001 bis 10 Gew.-% Trimethylcyclohexylamin (TMCHA) der Formel (I) 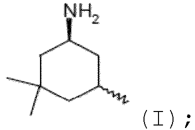
0,001 bis 10 Gew.-% an einer Ringverbindung (Ring) der Formel (II) 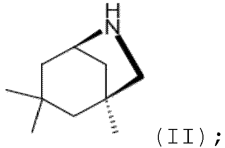
0,001 bis 10 Gew.-% Aminomethylmethylcyclohexylamin (AM-MCHA) der Formel (III); 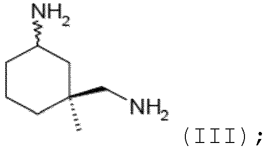 und
70 bis 99,997 Gew.-% Wasser.

14. Verfahren nach wenigstens einem der Ansprüche 1 bis 13, wobei Fraktion (ii) weniger als 0,01 Gew.-% HCN- oder Nitrilverbindungen umfasst.

15. Verfahren nach wenigstens einem der Ansprüche 1 bis 14, wobei der Vorbehandlungsschritt eine Kombination aus einem Stripping-Vorbehandlungsschritt und einem Adsorptions-Vorbehandlungsschritt oder eine Kombination aus einem Stripping-Vorbehandlungsschritt und einem Oxidations-Vorbehandlungsschritt ist.

16. Verfahren nach Anspruch 1, wobei Fraktion (ii) einem biologischen Abfallbehandlungsschritt unterzogen wird, nachdem sie einem oder mehreren Abwasser-Vorbehandlungsschritten ausgewählt aus der Gruppe bestehend aus Verdampfung, Oxidation und Adsorption unterzogen worden ist.

## Revendications

1. Procédé de production d'IPDA comprenant les étapes de :
I. conversion d'IPN à l'aide d'ammoniac et d'hydrogène en IPDA,
II. élimination de l'hydrogène et/ou de l'ammoniac de l'effluent de l'étape I
III. séparation de l'effluent de l'étape II en une ou plusieurs étapes en
(i) une fraction comprenant une phase organique ayant un point d'ébullition inférieur à celui de l'IPDA
(ii) une fraction comprenant une phase aqueuse ayant un point d'ébullition inférieur à celui de l'IPDA ;
(iii) une fraction comprenant de l'IPDA
(iv) une fraction comprenant des composants ayant un point d'ébullition supérieur à celui de l'IPDA ; et
IV. la soumission de la fraction (ii) à une ou plusieurs étapes de prétraitement des eaux usées, choisies parmi une évaporation, une oxydation et une adsorption.

2. Procédé selon la revendication 1, dans lequel l'une des une ou plusieurs étapes de prétraitement IV est une étape de prétraitement par extraction au gaz.

3. Procédé selon au moins l'une des revendications 1 à 2, dans lequel l'étape de prétraitement par extraction au gaz est réalisée dans une colonne d'extraction au gaz avec de la vapeur d'eau en tant que gaz d'extraction au gaz.

4. Procédé selon au moins l'une des revendications 1 à 3, dans lequel le condensat obtenu au sommet de la colonne d'extraction au gaz est séparé en une phase organique et une phase aqueuse.

5. Procédé selon au moins l'une des revendications 1 à 4, dans lequel la phase organique et/ou la phase aqueuse sont recyclées vers un séparateur de phases utilisé pour séparer les fractions (i) et (ii) après élimination des composants ayant un point d'ébullition inférieur à celui de l'IPDA de l'effluent de l'étape II.

6. Procédé selon au moins l'une des revendications 3 à 5, dans lequel au moins une partie de l'eau prétraitée obtenue à la base de la colonne d'extraction au gaz est utilisée pour chauffer directement ou indirectement la charge de fraction (ii) dans la colonne d'extraction au gaz.

7. Procédé selon au moins l'une des revendications 1 à 6, dans lequel la vapeur utilisée en tant que gaz d'extraction au gaz est (i) générée dans un rebouilleur raccordé au collecteur de la colonne d'extraction au gaz ou (ii) est fournie par un réseau de vapeur d'une installation chimique intégrée.

8. Procédé selon au moins l'une des revendications 1 à 7, dans lequel l'une des une ou plusieurs étapes de prétraitement IV est une étape de prétraitement par oxydation.

9. Procédé selon la revendication 8, dans lequel le rapport molaire de l'oxydant ajouté à l'impureté organique est dans la plage de 10:1 et 100:1.

10. Procédé selon au moins l'une des revendications 8 à 9, dans lequel l'oxydant chimique est le peroxyde d'hydrogène.

11. Procédé selon au moins l'une des revendications 1 à 10, dans lequel l'une des une ou plusieurs étapes de prétraitement IV est une étape de prétraitement par adsorption.

12. Procédé selon la revendication 11, dans lequel l'adsorbant est du charbon actif.

13. Procédé selon au moins l'une des revendications 1 à 13, dans lequel la fraction (ii) comprend
0,001 à 10 pour cent en poids de triméthylcyclohexylamine (TMCHA) de formule (I) 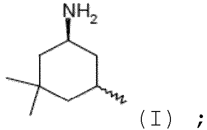
0,001 à 10 pour cent en poids d'un composé cyclique (Cycle) de formule (II) 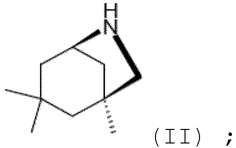
0,001 à 10 pour cent en poids d'aminométhyl-méthylcyclohexylamine (AM-MCHA) de formule (III) ; 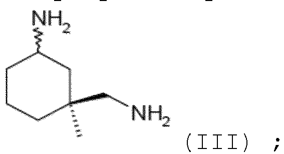 et
70 à 99,997 pour cent en poids d'eau.

14. Procédé selon au moins l'une des revendications 1 à 13, dans lequel la fraction (ii) comprend moins de 0,01 pour cent en poids de HCN ou de composés nitriles.

15. Procédé selon au moins l'une des revendications 1 à 14, dans lequel l'étape de prétraitement est une combinaison d'une étape de prétraitement par extraction au gaz et d'une étape de prétraitement par adsorption, ou une combinaison d'une étape de prétraitement par extraction au gaz et d'une étape de prétraitement par oxydation.

16. Procédé selon la revendication 1, dans lequel la fraction (ii) est soumise à une étape de traitement des déchets biologiques après avoir été soumise à une ou plusieurs étapes de prétraitement des eaux usées, choisies parmi une évaporation, une oxydation et une adsorption.
